# EUROPÄISCHE PATENTSCHRIFT

(11) **EP 0 434 833 B1**
(45) Veröffentlichungstag und Bekanntmachung des Hinweises auf die Patenterteilung: **25.05.1994**
(21) Anmeldenummer: 90917919.4
(22) Anmeldetag: 13.07.1990
(51) Int. Cl.: C07C 405/00, A61K 31/557

(54) **9-FLUOR-PROSTAGLANDIN-DERIVATE, VERFAHREN ZU IHRER HERSTELLUNG UND IHRE PHARMAZEUTISCHE VERWENDUNG**
9-FLUOR-PROSTAGLANDINE DERIVATES, THEIR PROCESS OF PRODUCTION AND THEIR PHARMACEUTICAL USE
DERIVES DE 9-FLUOR-PROSTAGLANDINE, LEUR PROCEDE DE PRODUCTION ET LEUR UTILISATION PHARMACEUTIQUE

(30) Priorität: 14.07.1989 DE 3923797
(43) Veröffentlichungstag der Anmeldung: 03.07.1991
(73) Patentinhaber: SCHERING AKTIENGESELLSCHAFT, 13342 Berlin (DE)
(72) Erfinder: KLAR, Ulrich, D-1000 Berlin 27 (DE); REHWINKEL, Hartmut, D-1000 Berlin 44 (DE); VORBRÜGGEN, Helmut, D-1000 Berlin 27 (DE); THIERAUCH, Karl-Heinz, D-1000 Berlin 37 (DE); STÜRZEBECHER, Claus-Steffen, D-1000 Berlin 19 (DE)
(86) Internationale Anmeldenummer: DE9000538
(87) Internationale Veröffentlichungsnummer: WO9101302

(56) Entgegenhaltungen:
- EP-A- 0 069 696
- EP-A- 0 299 914
- DE-A- 3 504 044

## Beschreibung

Die Erfindung betrifft 9-Fluor-prostaglandin-Derivate, Verfahren zu ihrer Herstellung sowie ihre Verwendung als Hilfsstoffe für pharmakologische Untersuchungen und als Arzneimitel.
Es wurde überraschenderweise gefunden, daß durch die Einführung eines Fluoratomes in die Position 9 in Verbindung mit einem aromatischen Substituenten an Position 17 chemisch und metabolisch stabile Prostaglandin-Analoga erhalten werden, deren pharmakologische Eigenschaften mit denen des instabilen Thromboxan-A₂(TXA₂) bzw. PGH₂ vergleichbar sind, während die in EP-A-69696 strukturell ähnlichen Prostaglandinderivate abortive und luteolytische Wirksamkeit zeigen.

Die Verbindungen dieser Erfindung eignen sich deshalb als Hilfsmittel für pharmakologische Charakterisierungen sowie zur selektiven Therapie von Erkrankungen, die auf einen Mangel an körpereigenem TXA₂/PGH₂ zurückzuführen sind.

Die Erfindung betrifft 9-Fluor-prostaglandin-Derivate der Formel I, 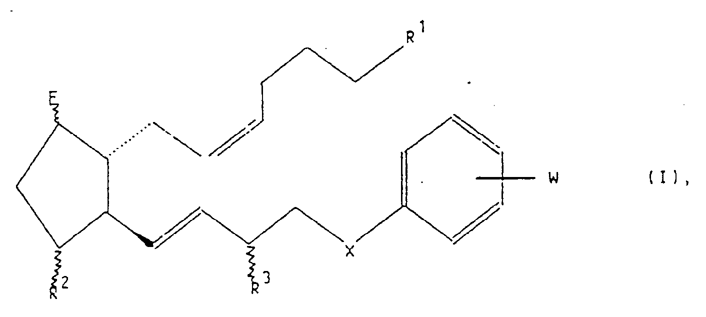
worin 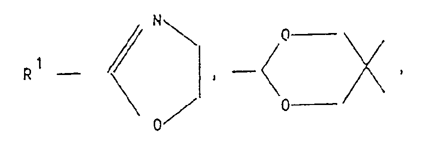
COOR⁴, wobei R⁴ Wasserstoff
oder einen gegebenenfalls durch Halogene, Hydroxy, C₁₋₄-Alkoxy, C₆₋₁₂-Aryl, das durch Halogen substituiert sein kann, Di-(C₁₋₄)-Alkylamino oder Tri-(C₁₋₄)-Alkylammonium substituierten C₁₋₁₀-Alkylrest, einen gegebenenfalls mit C₁₋₄-Alkyl substituierten C₃₋₁₀-Cycloalkylrest, einen C₇₋₁₆-Aralkylrest, einen durch W substituierten Phenacylrest, einen gegebenenfalls durch Halogen, Phenyl, C₁₋₄-Alkyl, Chlormethyl, Fluormethyl, Carboxyl, C₁₋₄-Alkoxy oder Hydroxy substituierten C₆₋₁₂-Arylrest oder einen 5- oder 6-gliedrigen heterocyclischen Rest mit wenigstens einem N-, O- oder S-Atom bedeuten kann, oder R¹ ein CONHR⁵-Rest mit R⁵ in der Bedeutung Wasserstoff, C₁-C₁₀-Alkanoyl oder C₁-C₁₀-Alkansulfonyl sein kann,
- R² und R³: jeweils ein Wasserstoffatom oder eine freie oder funktionell abgewandelte Hydroxygruppe, wobei die OH-Gruppe jeweils α- oder β-ständig sein kann, bedeuten,
- X: eine CH₂-Gruppe, ein O- oder S-Atom,
- W: Wasserstoff, -OR⁶, Halogen, -CN- , -NO₂, Trifluormethyl oder COOR⁶,

R⁶ Wasserstoff, einen gegebenenfalls durch Halogene, Hydroxy, C₁₋₄-Alkoxy, C₆₋₁₂-Aryl, das durch Halogen substituiert sein kann, Di-(C₁₋₄)-Alkylamino oder Tri-(C₁₋₄)-Alkylammonium substituierten C₁₋₁₀-Alkylrest, einen gegebenenfalls durch Halogen, Phenyl, C₁₋₄-Alkyl, Chlormethyl, Fluormethyl, Carboxyl, C₁₋₄-Alkoxy oder Hydroxy substituierten C₆₋₁₂-Arylrest oder
C₇-C₁₆-Aralkyl sein kann und, falls R⁴ Wasserstoff bedeutet, deren Salze mit physiologisch verträglichen Basen, sowie die α-, β- oder γ-Cyclodextrinclathrate, sowie die mit Liposomen verkapselten Verbindungen der Formel I bedeuten.

Die Definition 5- oder 6-gliedriger heterocyclischer Rest betrifft Heterocyclen, die wenigstens ein Heteroatom, vorzugsweise Stickstoff, Sauerstoff oder Schwefel enthalten. Beispielsweise seien genannt 2-Furyl, 3-Furyl, 2-Thienyl, 3-Thienyl, 2-Pyridyl, 3-Pyridyl, 4-Pyridyl.
Als Alkylgruppen R⁴ and R⁶ sind gerad- oder verzweigtkettige Alkylgruppen mit 1-10 C-Atomen zu betrachten, wie beispielsweise Methyl, Ethyl, Propyl, Isopropyl, Butyl, Isobutyl, tert.-Butyl, Pentyl, Isopentyl, Neopentyl, Heptyl, Hexyl, Decyl.

Die Alkylgruppen R⁴ und R⁶ können substituiert sein durch Halogenatome, Hydroxygruppen, C₁-C₄-Alkoxygruppen, C₆-C₁₂-Arylgruppen, die durch Halogen substituiert sein können, Di-(C₁-C₄)-Alkylamine und Tri-(C₁-C₄)-Alkylammonium. Bevorzugt sind solche Alkylgruppen, die einfach substituiert sind.

Als Substituenten seien beispielsweise genannt Fluor-, Chlor- oder Bromatome, Phenyl, Dimethylamino, Diethylamino, Methoxy, Ethoxy.

Als bevorzugte Alkylgruppen R⁴ und R⁶ sind solche mit 1-4 C-Atomen, wie zum Beispiel Methyl, Ethyl, Propyl, Isobutyl, Butyl, zu nennen.

Als Arylgruppen R⁴ und R⁶ kommen beispielsweise in Betracht: Phenyl, Diphenyl, 1-Naphthyl und 2-Naphthyl, die substituiert sein können durch 1-3 Halogenatome, eine Phenylgruppe, 1-3 Alkylgruppen mit jeweils 1-4 C-Atomen, eine Chlormethyl-, Fluormethyl-, Carboxyl-, C₁-C₄-Alkoxy- oder Hydroxygruppe. Bevorzugt ist die Substitution in 3- und 4-Stellung am Phenylring zum Beispiel durch Fluor, Chlor, C₁-C₄-Alkoxy oder Trifluormethyl oder in 4-Stellung durch Hydroxy.

Die Cycloalkylgruppen R⁴ können im Ring 3-10, vorzugsweise 3-6 Kohlenstoffatome enthalten. Die Ringe können durch Alkylgruppen mit 1-4 Kohlenstoffatomen substituiert sein. Beispielsweise seien genannt Cyclopropyl, Cyclobutyl, Cyclopentyl, Methylcyclopentyl, Methylcyclohexyl.
Besonders bevorzugte Cycloalkylgruppen sind Cyclopentyl und Cyclohexyl. Als C₇-C₁₆-Aralkyl sind folgende Reste gemeint: Phenyl-substituierte Alkylreste (geradkettige und verzweigte) mit 1-10 C-Atomen wie zum Beispiel Benzyl, Phenylmethyl, α-Phenylethyl, 3-Phenylpropyl usw. Als Ar kommen aber auch 1-oder 2-Naphthyl mit entsprechend kürzerer Alkylkette in Betracht.

Die als Substituenten genannten Alkyl- oder Alkoxygruppen mit 1-4 C-Atomen sollen gerad- oder verzweigtkettig sein.

Die Hydroxygruppen in R² und R³ können funktionell abgewandelt sein, beispielsweise durch Veretherung oder Veresterung, wobei die freien oder abgewandelten Hydroxygruppen α- oder β-ständig sein können, wobei freie Hydroxygruppen bevorzugt sind.

Als Ether- und Acylreste kommen die dem Fachmann bekannten Reste in Betracht. Bevorzugt sind leicht abspaltbare Etherreste, wie beispielsweise der Tetrahydropyranyl- , Tetrahydrofuranyl-, tert.-Butyldimethylsilyl-, tert.-Butyldiphenylsilyl-, Tribenzylsilylrest. Als Acylreste kommen zum Beispiel Acetyl, Propionyl, Butyryl, Benzoyl in Frage.

Halogen in den Definitionen für R⁴, R⁶ und W bedeutet Fluor, Chlor und Brom.

Die Reste "C₁-C₁₀-Alkanoyl" oder "C₁-C₁₀-Alkansulfonyl" für R⁵ entsprechen den bereits genannten Alkylgruppen gleicher Länge mit dem Unterschied, daß sie an eine Carboxylgruppe gebunden sind. Bevorzugt sind C₁-C₄-Alkanoyl bzw. -Alkansulfonyl.

Zur Salzbildung mit den freien Säuren (R⁴ = H) sind anorganische und organische Basen geeignet, wie sie dem Fachmann zur Bildung physiologisch verträglicher Salze bekannt sind. Beispielsweise seien genannt: Alkalihydroxide, wie Natrium- oder Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroymethyl)-methylamin usw.
Bevorzugte Verbindungen der Formel I sind Verbindungen in denen
- R¹: die Gruppe COOR⁴,
- R²: Wasserstoff oder Hydroxyl,
- R³: Wasserstoff oder Hydroxyl,
- R⁴: Wasserstoff oder C₁-C₆-Alkyl,
- R⁵: Methansulfonyl,
- X: Sauerstoff oder CH₂,
- W: Wasserstoff oder Fluor bedeuten.

Die Erfindung betrifft ferner ein Verfahren zur Herstellung der Verbindungen der Formel I, das dadurch gekennzeichnet ist, daß man eine Verbindung der Formel II 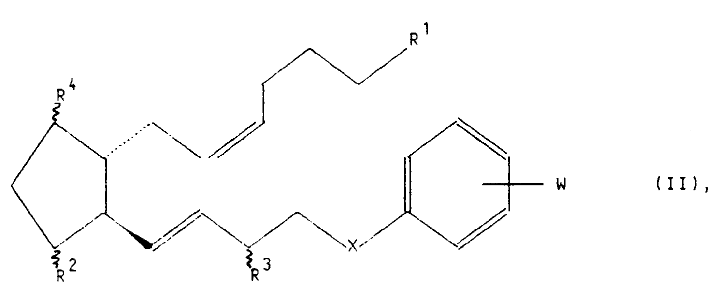
worin
R⁴ eine Hydroxygruppe und R¹, R², R³, X und W die oben angegebenen Bedeutungen aufweisen und freie OH-Gruppen in R², R³ und W geschützt sind, mit Diethylaminoschwefeltrifluorid [M. Sharma, Tetrahedron Lett. 573 (1977); W.J. Middleton, J. Org. Chem. 40, 574(1975)] oder anderen Fluorierungsmitteln wie z.B.(HF)ₙ.-Pyridin [G.A. Olah, Synthesis 786(1973)] oder SeF₄.Pyridin [G.A. Olah, J. Am. Chem. Soc. 96, 925 (1974)] umsetzt und gegebenenfalls geschützte Hydroxygruppen in R², R³ und W freisetzt und/oder freie Hydroxygruppen verestert, verethert und/oder eine veresterte Carboxygruppe verseift oder eine Carboxygruppe mit einer physiologisch verträglichen Base in ein Salz überführt oder mit α-, β- oder γ-Cyclodextrin zu einem Clathrat umsetzt oder mit Liposomen verkapselt.

Die Umsetzung der Verbindungen der allgemeinen Formel II zu den Verbindungen der allgemeinen Formel I wird mit Diethylaminoschwefeltrifluorid bei -80°C bis +40°C, vorzugsweise bei -70°C bis +25°C durchgeführt. Als Lösungsmittel eignen sich Dichlormethan, 1.1.2-Trifluortrichlorethan, Pyridin, Toluol, Benzol, Ethylenchlorid u.a., vorzugsweise Toluol und Pyridin.
Die Freisetzung der funktionell abgewandelten Hydroxygruppen R², R³ und W erfolgt nach den dem Fachmann bekannten Methoden. Beispielsweise wird die Abspaltung von Etherschutzgruppen in einer wäßrigen Lösung einer organischen Säure, wie z.B. Essigsäure, Propionsäure, Zitronensäure u.a. oder in einer wäßrigen Lösung einer anorganischen Säure, wie z.B. Salzsäure, oder im Falle von Tetrahydropyranylethern unter Verwendung von Pyridinium-p-Toluolsulfonat, vorzugsweise in Alkoholen als Lösungsmittel oder unter Verwendung von wasserfreiem Magnesiumbromid, vorzugsweise in Diethylether als Lösungsmittel durchgeführt.

Zur Verbesserung der Löslichkeit wird bei Verwendung wäßrig-saurer Reaktionsbedingungen zweckmäßigerweise ein mit Wasser mischbares inertes Lösungsmittel zugesetzt. Als geeignet erweisen sich z.B. Alkohole wie Methanol und Ethanol, Ether wie Dimethoxyethan, Dioxan und Tetrahydrofuran, wobei Tetrahydrofuran bevorzugt angewendet wird.

Die Abspaltung der Silyletherschutzgruppen erfolgt beispielsweise mit Tetrabutylammoniumfluorid nach den dem Fachmann bekannten Methoden. Als Lösungsmittel sind beispielsweise Tetrahydrofuran, Diethylether, Dioxan, Methylenchlorid, usw. geeignet. Die Abspaltung wird vorzugsweise bei Temperaturen zwischen 20° und 80°C durchgeführt.

Die Verseifung der Acylgruppen und Prostaglandinester wird nach den dem Fachmann bekannten Methoden durchgeführt., wie beispielsweise mit basischen Katalysatoren wie z.B. mit Alkali- oder Erdalkali-carbonaten oder -hydroxiden in einem Alkohol oder der wäßrigen Lösung eines Alkohols. Als Alkohole kommen aliphatische Alkohole wie z.B. Methanol, Ethanol, Butanol usw. in Betracht, vorzugsweise jedoch Methanol. Als Alkalicarbonate und -hydroxide seien Lithium-, Natrium- und Kaliumsalze genannt. Bevorzugt sind die Lithium- und Kaliumsalze. Als Erdalkalicarbonate und -hydroxide eignen sich beispielsweise Calciumcarbonat, Calciumhydroxid und Bariumcarbonat. Die Umsetzung erfolgt allgemein bei -10° bis +70°C, vorzugsweise jedoch bei +25°C.

Die Einführung der Estergruppen CO₂R⁴ für R¹ bzw. CO₂R⁶ für W, bei welcher R⁴ bzw. R⁶ eine Alkylgruppe mit 1-10 C-Atomen darstellt, erfolgt nach den dem Fachmann bekannten Methoden. Die 1-Carboxyverbindungen (R⁴ = H bzw. R⁶ = H) werden beispielsweise mit Diazokohlenwasserstoffen in an sich bekannter Weise umgesetzt. Die Veresterung mit Diazokohlenwasserstoffen erfolgt z.B. dadurch, daß eine Lösung des Diazokohlenwasserstoffes in einem inerten Lösungsmittel, vorzugsweise in Diethylether, mit der 1-Carboxyverbindung, gelöst in dem gleichen oder in einem anderen inerten Lösungsmittel, wie z.B. Methylenchlorid, vermischt wird. Nach beendeter Umsetzung in 1 bis 60 Minuten wird das Lösungsmittel entfernt und der Ester in üblicher Weise gereinigt. Diazoalkane sind entweder bekannt oder können nach bekannten Methoden hergestellt werden (Org. Reactions Bd. 8, Seiten 389-394(1954)).

Die Einführung der Estergruppe CO₂R⁴ für R¹ bzw. CO₂R⁶ für W, bei welcher R⁴ bzw. R⁶ eine substituierte oder unsubstituierte Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base wie z.B. Pyridin, Dimethylaminopyridin, Triethylamin, in einem inerten Lösungsmittel wie z.B. Methylenchlorid, Ethylenchlorid, Chloroform, Essigsäureethylester, Tetrahydrofuran, vorzugsweise jedoch mit Chloroform umgesetzt. Die Reaktion wird bei Temperaturen zwischen -30°C und +50°C, vorzugsweise bei +10°C, durchgeführt.

Die Prostaglandinderivate der Formel I mit R⁴ bzw. R⁶ in der Bedeutung eines Wasserstoffatomes können mit geeigneten Mengen der entsprechenden anorganischen Basen unter Neutralisierung in Salze überführt werden. Beispielsweise erhält man beim Lösen der entsprechenden Prostaglandinsäuren in Wasser, welches stöchiometrische Mengen der Base enthält, nach Abdampfen des Wassers oder nach Zugabe eines mit Wasser mischbaren Lösungsmittels, z.B. Alkohol oder Aceton, das feste anorganische Salz.

Die Herstellung der Aminsalze erfolgt in üblicher Weise. Dazu löst man die Prostaglandinsäure in einem geeigneten Lösungsmittel, wie z.B. Ethanol, Aceton, Diethylether oder Benzol und setzt 1 bis 5 Äquivalente des jeweiligen Amins dieser Lösung zu. Dabei fällt das Salz gewöhnlich in fester Form an oder wird nach Verdampfen des Lösungsmittels in üblicher Weise isoliert.

Die funktionelle Abwandlung der freien Hydroxygruppen erfolgt nach den dem Fachmann bekannten Methoden. Zur Einführung der Esterschutzgruppen wird beispielsweise mit Dihydropyran oder Methylvinylether in Methylenchlorid oder Chloroform unter Verwendung katalytischer Mengen eines sauren Kondensationsmittels wie z.B. Toluolsulfonsäure, umgesetzt. Der jeweilige Enolether wird im Überschuß, vorzugsweise in der 1,2- bis 10-fachen Menge des theoretischen Bedarfs, zugesetzt. Die Umsetzung erfolgt normalerweise bei -10°C bis +30°C und ist nach 2 bis 45 Minuten beendet.

Zur Einführung von Silyletherschutzgruppen wird beispielsweise mit t-Butyl-diphenylchlorsilan oder t-Butyl-dimethylchlorsilan in Dimethylformamid unter Verwendung einer Base wie z.B. Imidazol, umgesetzt. Das jeweilige Silylchlorid wird im Überschuß, vorzugsweise in der 1,05- bis 4-fachen Menge des theoretischen Bedarfs, zugesetzt. Die Umsetzung erfolgt normalerweise bei 0°C bis 30°C und ist nach 1 bis 24 Stunden beendet.

Die Einführung der Acylschutzgruppen erfolgt, indem man eine Verbindung der Formel I in an sich bekannter Weise mit einem Carbonsäurederivat, wie z.B. Säurechlorid, Säureanhydrid usw., umsetzt.

Die neuen chemisch und metabolisch stabilen 9-Fluor-prostaglandin-Derivate besitzen pharmakologische Eigenschaften, die denen des instabilen Thromboxan-A₂(TXA₂) bzw. PGH₂ vergleichbar sind. Sie stellen als TXA₂/PGH₂-Rezeptoragonisten somit ein wertvolles diagnostisches Instrument zur Charakterisierung von Prostaglandinrezeptoren bzw. TXA₂/PGH₂-Rezeptorsubtypen, mit denen sich die Bedeutung der TXA₂/PGH₂-abhängigen Stimulation von Plättchen und Gefäßen nachweisen läßt, dar. Dies gilt sowohl für in vitro Tests wie z.B. Rezeptorcharakterisierung bzw. Verdrängung am Rezeptor, Thrombozytenaggregationshemmteste, Gefäßstreifenkonstriktion usw., als auch für pharmakologische Untersuchungen am Tier.

Die TXA₂/PGH₂-Rezeptoragonisten können zur spezifischen Abschwächung oder Aufhebung der Wirkung von Cyclooxygenasehemmstoffen, von TXA₂-Synthetasehemmstoffen sowie von TXA₂/PGH₂-Rezeptorblockern verwendet werden. Eine weitere Einsatzmöglichkeit besteht in der partiellen Herunterregulation der TXA₂/PGH₂-Wirkung bei Krankheitsbildern mit erhöhter Sensitivität gegenüber bzw. Produktion von Thromboxan wie z.B. die von Koronarien oder Gefäßen mit arteriosklerotischen Läsionen.

In Kombination mit einem TXA₂/PGH₂-Rezeptorantagonisten läßt sich der TXA₂/PGH₂-Rezeptoragonist zur diagnostischen Abklärung der Beteiligung TXA₂/PGH₂-abhängiger Prozesse bei solchen Krankheitsbildern verwenden, die zu dieser Diagnostik keine systemische Gabe eines TXA₂/PGH₂-Rezeptoragonisten fordern, aber auch bei anderen Krankheitsbildern, sofern unerwünschten Wirkungen des TXA₂/PGH₂-Rezeptoragonisten durch einen Antagonisten gegengesteuert werden kann.

Die TXA₂/PGH₂-Rezeptoragonisten sind weiterhin geeignet zur lokalen Blutstillung bei Defekten der Plättchenfunktion, die auf einer Störung der TXA₂/PGH₂-Bildung und/oder Wirkung basieren.

Die Fluorprostaglandin-Derivate dieser Erfindung können auch in Kombination, z.B. mit β-Blockern, Diuretika, Phosphodiesterasehemmern, Ca-Antagonisten oder nichtsteroidalen Entzündungshemmern, verwendet werden.

Die Dosis der Verbindungen ist 1-1000µg/kg/Tag, wenn sie am menschlichen Patienten verabreicht wird. Die Einheitsdosis für den pharmazeutischen akzeptablen Träger beträgt 10 µg bis 100 µg.

Für die parenterale Applikation werden sterile, injizierbare wäßrige oder ölige Lösungen benutzt. Für die orale Applikation sind beispielsweise Tabletten, Dragees oder Kapseln geeignet. Die Erfindung betrifft somit auch Arzneimittel auf Basis der Verbindungen der Formel I und üblicher Hilfs- und Trägerstoffe einschließlich Cyclodextrinclathrate und Verkapselung von Liposomen.

Die erfindungsgemäßen Wirkstoffe sollen in Verbindung mit den in der Galenik bekannten und üblichen Hilfsstoffen zum Beispiel zur Herstellung von Arzneimitteln dienen.

### BEISPIEL 1

(5Z,9R,13E,15R)-9-Fluor-15-hydroxy-16-phenoxy-17,18,19,20-tetranor-5,13-prostadiensäuremethylester:

Die Lösung von 47 mg (75 µmol) (5Z,9R,13E,15R)-9-Fluor-15-t-butyl-diphenylsi lyloxy-16-phenoxy-17,18,19,20-tetranor-5,13-prostadiensäuremethylester [vergl. Dr. U. Klar et al., Deutsche Patentanmeldung, Aktenzeichen P3923798.2 löst man in 870µl wasserfreiem Tetrahydrofuran, versetzt mit 170µl eine 1M Tetrabutylammoniumfluoridlösung in Tetrahydrofuran und läßt drei Stunden unter einer Atmosphäre aus trockenem Argon rühren. Man gießt auf Eiswasser, extrahiert mit Diethylether, wäscht mit einer gesättigten Natriumchloridlösung nach und trocknet über Magnesiumsulfat. Das nach Lösungsmittelabzug im Wasserstrahlvakuum erhaltene Rohöl reinigt man durch Chromatographie an zwei analytischen Dünnschichtplatten. Als Laufmittel dient ein Gemisch aus n-Hexan und Ethylacetat, als Elutionsmittel Diethylether. Man isoliert 26 mg (67 µmol, 89%) der Titelverbindung als farbloses Öl.

IR (Film): 3600-3200, 3060, 3030, 3010, 2930, 2870, 1735, 1600, 1585, 1495, 1245, 1040, 970, 750 und 690 cm⁻¹.

### BEISPIEL 2

(5Z,9R,13E,15R)-9-Fluor-15-hydroxy-16-phenoxy-17,18,19,20-tetranor-5,13-prostadiensäure:

26 mg (67 µmol) der in Beispiel 1 dargestellten Verbindung löst man in 970 µl Methanol, versetzt mit 324 µl einer 8%igen Kaliumhydroxidlösung und rührt 5 Stunden bei 25°C. Man gießt in Eiswasser, stellt durch Zugabe einer gesättigten Zitronensäurelösung einen pH-Wert von 4 bis 5 ein, extrahiert mehrfach mit Dichlormethan, wäscht mit Wasser und trocknet über Magnesiumsulfat. Isoliert werden 25 mg (66 µmol, 99%) der Titelverbindung als farbloses Öl.

IR (Film): 3600-2400, 3060, 3030, 3010, 2930, 2870, 1710, 1600, 1585, 1495, 1245, 1040, 970, 755 und 690 cm⁻¹.

### BEISPIEL 3

(5Z,9R,13E,15S)-9-Fluor-15-hydroxy-16-phenoxy-17,18,19,20-tetranor-5,13-prostadiensäuremethylester:

41 mg (65 µmol) (5Z,9R,13E,15S)-9-Fluor-15-t-butyl-diphenylsilyloxy-16 -phenoxy-17,18,19,20-tetranor-5,13-prostadiensäuremethylester [vergl. Dr. U. Klar et al., Deutsche Patentanmeldung, Aktenz. P3923798.2 setzt man in Analogie zu Beispiel 1 um und isoliert nach Aufarbeitung und Reinigung 25 mg (64 µmol, 98%) der Titelverbindung als farbloses Öl.
IR (Film): 3600-3200, 3060, 3020, 2940, 2870, 1740, 1600, 1590, 1245, 1040, 970, 755 und 695 cm⁻¹.

### BEISPIEL 4

(5Z,9R,13E,15S)-9-Fluor-15-hydroxy-16-phenoxy-17,18,19,20-tetranor-5,13-prostadiensäure:

25 mg (64 µmol) der in Beispiel 3 dargestellten Verbindung setzt man in Analogie zu Beispiel 2 um und isoliert nach Aufarbeitung 24 mg (64 µmol, 100%) der Titelverbindung als farbloses Öl.

IR (Film): 3600-2400, 3060, 3010, 2940, 2870, 1710, 1600, 1590, 1500, 1245, 1080, 1040, 970, 755 und 690 cm⁻¹.

### BEISPIEL 5

(5Z,9R,11S,13E,15R)-9-Fluor-11,15-dihydroxy-16-phenoxy-17,18,19,20-tetranor-5,13-prostadiensäure:

54 mg (106 µmol) der in Beispiel 5a dargestellten Verbindung verseift man in Analogie zu Beispiel 2 und isoliert nach Aufarbeitung und Reinigung 36 mg (92 µmol, 87%) der Titelverbindung als farbloses Öl.

IR (Film): 3600-2400, 3090, 3060, 3040, 3010, 2930, 2870, 1710, 1600, 1585, 1495, 1245, 1085, 1040, 975, 755 und 690 cm⁻¹.
a) (5Z,9R,11S,13E,15R)-9-Fluor-11-benzoyloxy-15-hydroxy-16-phenoxy-17,18,19,20-tetranor-5,13-prostadiensäuremethylester:
   86 mg (115µmol) der in Beispiel 5b dargestellten Verbindung setzt man in Analogie zu Beispiel 1 um und isoliert nach Aufarbeitung und Reinigung 54 mg (106 µmol, 92%) der Titelverbindung als farbloses Öl.
   IR (Film): 3600-3200, 3060, 3020, 2950, 2870, 1735, 1710, 1600, 1495, 1445, 1245, 1110, 1065, 1025, 970, 755, 715 und 695 cm⁻¹.
b) (5Z,9R,11S,13E, 15R)-9-Fluor-11-benzoyloxy-15-t-butyl-diphenylsilyloxy-16-phenoxy-17,18,19,20-tetranor-5,13-prostadiensäuremethylester:
   78 mg (121 µmol) der in Beispiel 5c dargestellten Verbindung löst man in 3 ml wasserfreiem Toluol, versetzt mit 65 mg Triphenylphosphin, 30 mg Benzoesäure und 39 µl Azodicarbonsäurediethylester (DEAD). Man rührt 2,5 Stunden bei 25°C unter einer Atmosphäre aus trockenem Argon, versetzt mit Wasser, extrahiert mehrfach mit Diethylether, trocknet über Magnesiumsulfat und reinigt den nach Lösungsmittelabzug erhaltenen Rückstand durch Chromatographie an ca. 30 ml feinem Kieselgel. Als Laufmittel dient ein Gemisch aus Ethylacetat und n-Hexan. Isoliert werden 86 mg (115 µmol, 95%) der Titelverbindung als farbloses Öl.
   IR (Film): 3070, 3020, 3000, 2940, 2860, 1735, 1715, 1600, 1590, 1490, 1445, 1425, 1270, 1245, 1110, 970, 820, 745 und 700 cm⁻¹.
c) (5Z,9R,11R,13E,15R)-9-Fluor-11-hydroxy-15-t-butyl-diphenylsilyloxy-16-Phenoxy-17,18,19,20-tetranor-5,13-prostadiensäuremethylester:
   93 mg (128 µmol) (5Z,9R,11R,13E,15R)-9-Fluor-11-(tetrahydropyran-2-yloxy)-15-t-butyldiphenylsilyloxy-16-phenoxy-17,18,19,20-tetranor-5,8(9),13- prostatriensäuremethylester [vergl. Dr. U. Klar et al., Deutsche Patentanmeldung, Aktenz. P3923798.2 löst man in 2 ml wasserfreiem Methanol, versetzt mit 11 mg Pyridinium-p-Toluolsulfonat (PPTs) und erwärmt unter einer Atmosphäre aus trockenem Argon 2 Stunden auf 55°C. Nach dem Erkalten versetzt man mit Dichlormethan, wäscht mit Wasser und gesättigter Natriumchloridlösung, trocknet über Magnesiumsulfat und reinigt den nach Lösungsmittelabzug erhaltenen Rückstand durch Chromatographie an vier analytischen Dünnschichtplatten. Als Laufmittel dient ein Gemisch aus n-Hexan und Ethylacetat, als Elutionsmittel Ethylacetat. Isoliert werden 78 mg (121 µmol, 95%) der Titelverbindung als farbloses Öl.
   IR (Film): 3600-3200, 3070, 3030, 3000, 2940, 2850, 1735, 1600, 1590, 1495, 1450, 1245, 1110, 970, 815, 750, 740 und 705 cm⁻¹.

### BEISPIEL 6

(5Z,9R,11S,13E,15R)-9-Fluor-11,15-dihydroxy-16-phenoxy-17,18,19,20-tetranor-5,13-prostadiensäuremethylester:

12 mg (31 µmol) der in Beispiel 5 dargestellten Säure löst man in 0,5 ml Dichlormethan, kühlt auf 5°C und versetzt mit einer etherischen Lösung von Diazomethan. Man läßt 15 Minuten rühren, entfernt überschüssiges Reagenz und Lösungsmittel durch Destillation im Wasserstrahlkvakuum und isoliert 12 mg (30 µmol, 97%) der Titelverbindung als farbloses Öl.

IR (Film): 3600-3200, 3090, 3060, 3030, 3010, 2930, 2870, 1735, 1600, 1590, 1495, 1245, 1085, 1040, 975, 760 und 690 cm⁻¹.

### BEISPIEL 7

(5Z,9R,11S,13E,15S)-9-Fluor-11,15-dihydroxy-16-phenoxy-17,18,19.20-tetranor-5,13-prostadiensäure:

139 mg (272 µmol) (5Z,9R,11S,13E,15S)-9-Fluor-11-benzoyloxy-15-hydroxy-16-phenoxy-17,18,19,20- tetranor-5,8),13-prostadiensäuremethylester [vergl. Dr. U. Klar et al., Deutsche Patentanmeldung,Aktenz. P3923798.2 verseift man in Analogie zu Beispiel 2 und isoliert nach Aufarbeitung und chromatographischer Reinigung 88 mg (224 µmol, 82%) der Titelverbindung als farbloses Öl.

IR (Film): 3600-2400, 3070, 3050, 3010, 2940, 2870, 1710, 1600, 1590, 1495, 1445, 1245, 1080, 1040, 980, 755 und 695 cm⁻¹.

### BEISPIEL 8

(5Z,9R,11S,13E,15R)-9-Fluor-11,15-dihydroxy-16-phenoxy-17,18,19,20-tetranor-5,13-prostadiensäuremethylester:

16 mg (41 µmol) der in Beispiel 7 dargestellten Verbindung verestert man in Analogie zu Beispiel 6 und isoliert nach Aufarbeitung und Reinigung 16 mg (39 µmol , 96%) der Titelverbindung als farbloses Öl.

IR (Film): 3600-3200, 3070, 3050, 3010, 2940, 2870, 1740, 1600, 1585 , 1500, 1245, 1080, 1040, 980, 755 und 695 cm⁻¹.

### BEISPIEL 9

(5Z,9R,11S,13E,15R)-9-Fluor-11,15-dihydroxy-17-phenyl-18,19,20-tri-nor-5,13-prostadiensäuremethylester:

360 mg (629 µmol) (5Z,9R,11R,13E,15R)-9-Fluor-11,15-bis-(tetrahydropyran-2-yloxy)-17-phenyl-18,19,20-tri-nor-5,13-prostadiensäuremethylester [vergl. Dr. U. Klar et al., Deutsche Patentanmeldung, Aktenz. P3923798.2 , versetzt man mit 15 ml eines Essig/Wasser/Tetrahydrofuran (65:35:10)-Gemisches und läßt 16 Stunden bei 23°C rühren. Man engt im Wasserstrahlvakuum ein und entfernt restliche Essigsäure azeotrop durch wiederholte Zugabe von Toluol. Man isoliert 238 mg (587 µmol, 93%) der Titelverbindung als farbloses Öl, das man ohne Reinigung weiter umsetzt.

IR (Film): 3600-3200, 3070, 3040, 3010, 2940, 2870, 1740, 1600, 1585, 1495, 1245, 1080, 1040, 980, 760 und 695 cm⁻¹.

### BEISPIEL 10

(5Z,9R,11R,13E,15R)-9-Fluor-11,15-dihydroxy-17-phenyl-18,19,20-tri-nor-5,13-prostadiensäure:

238 mg (587 µmol) der in Beispiel 9 dargestellten Verbindung setzt man in Analogie zu Beispiel 2 um und isoliert nach Aufarbeitung 176 mg (452 µmol, 77%) der Titelverbindung als farbloses Öl.

IR (KBr): 3600-2400, 3090, 3070, 3030, 3010, 2960, 2930, 2870, 1715, 1605, 1500, 1455, 1410, 1355, 1225, 1205, 1165, 1105, 1085, 1060, 1050, 980, 870, 825, 755 und 700 cm⁻¹.

### BEISPIEL 11

(5Z,9R,11R,13E,15S)-9-Fluor-11,15-dihydroxy-17-phenyl-18,19,20-tri-nor-5,13-prostadiensäuremethylester:

314 mg (549 µmol) (5Z,9R,11R,13E,15S)-9-Fluor-11,15-bis-(tetrahydropyran-2-yloxy)-17-phenyl-18,19,20-tri-nor-5,13-prostadiensäuremethylester [vergl. Dr. U. Klar et al., Deutsche Patentanmeldung,Aktenz. P3923798.2 setzt man in Analogie zu Beispiel 9 um und isoliert nach Aufarbeitung und Reinigung 221 mg (538 µmol, 98%) der Titelverbindung als farbloses Öl.

IR (Film): 3600-3200, 3080, 3060, 3030, 3010, 2940, 2860, 1735, 1600, 1495, 1450, 1410, 1245, 1085, 1045, 970, 750 und 700 cm⁻¹.

### BEISPIEL 12

(5Z,9R,11R,13E,15S)-9-Fluor-11,15-dihydroxy-17-phenyl-18,19,20-tri-nor-5,13-prostadiensäure:

221 mg (538 µmol) der in Beispiel 11 dargestellten Verbindung setzt man in Analogie zu Beispiel 2 um und isoliert nach Aufarbeitung 145 mg (372 µmol, 69%) der Titelverbindung als farbloses Öl.

IR (Film): 3600-2400, 3080, 3060, 3030, 3010, 2940, 2860, 1710, 1600, 1550, 1495, 1450, 1435, 1410, 1245, 1090, 1045, 1030, 970, 750 und 700 cm⁻¹.

## Patentansprüche

1. 9-Fluor-prostaglandin-Derivate der Formel I, 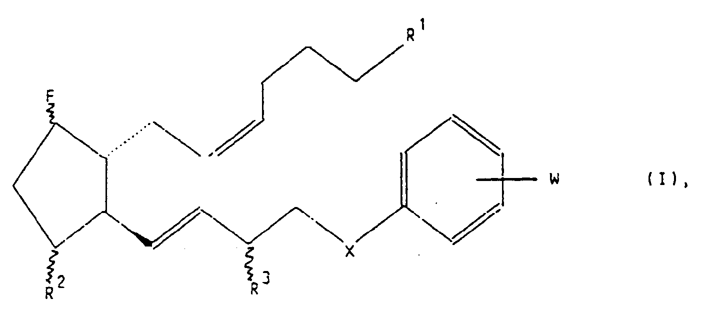 worin 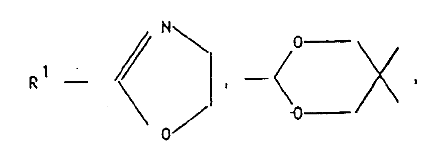 COOR⁴, wobei R⁴ Wasserstoff oder einen gegebenenfalls durch Halogene, Hydroxy, C₁₋₄-Alkoxy, C₆₋₁₂-Aryl, das durch Halogen substituiert sein kann, Di-(C₁₋₄)-Alkylamino oder Tri-(C₁₋₄)-Alkylammonium substituierten C₁₋₁₀-Alkylrest, einen gegebenenfalls mit C₁₋₄-Alkyl substituierten C₃₋₁₀-Cycloalkylrest, einen C₇₋₁₆-Aralkylrest, einen durch W substituierten Phenacylrest, einen gegebenenfalls durch Halogen, Phenyl, C₁₋₄-Alkyl, Chlormethyl, Fluormethyl, Carboxyl, C₁₋₄-Alkoxy oder Hydroxy substituierten C₆₋₁₂-Arylrest oder einen 5- oder 6-gliedrigen heterocyclischen Rest mit wenigstens einem N-, O- oder S-Atom bedeuten kann, oder R¹ ein CONHR⁵-Rest mit R⁵ in der Bedeutung Wasserstoff, C₁-C₁₀-Alkanoyl oder C₁-C₁₀-Alkansulfonyl sein kann,
R² und R³ jeweils ein Wasserstoffatom oder eine freie oder funktionell abgewandelte Hydroxygruppe, wobei die OH-Gruppe jeweils α- oder β-ständig sein kann, bedeuten,
X eine CH₂-Gruppe, ein O- oder S-Atom,
W Wasserstoff, -OR⁶, Halogen, -CN-, -NO₂, Trifluormethyl oder COOR⁶,
R⁶ Wasserstoff, einen gegebenenfalls durch Halogene, Hydroxy, C₁₋₄-Alkoxy, C₆₋₁₂-Aryl, das durch Halogen substituiert sein kann, Di-(C₁₋₄)-Alkylamino oder Tri-(C₁₋₄)-Alkylammonium substituierten C₁₋₁₀-Alkylrest, einen gegebenenfalls durch Halogen, Phenyl, C₁₋₄-Alkyl, Chlormethyl, Fluormethyl, Carboxyl, C₁₋₄-Alkoxy oder Hydroxy substituierten C₆₋₁₂-Arylrest oder
C₇-C₁₆-Aralkyl sein kann und, falls R⁴ Wasserstoff bedeutet, deren Salze mit physiologisch verträglichen Basen, sowie die α-, β- oder γ-Cyclodextrinclathrate, sowie die mit Liposomen verkapselten Verbindungen der Formel I bedeuten.

2. 9-Fluor-prostaglandin-Derivate der Formel I nach Anspruch 1, dadurch gekennzeichnet, daß
R¹ den Rest COOR⁴ mit R⁴ als Wasserstoff oder C₁-C₆-Alkyl oder R¹ den Rest CONHR⁵ mit R⁵ als Methylsulfonyl,
X eine CH₂-Gruppe oder ein O-Atom,
R² und R³ Wasserstoff oder Hydroxy und
W Wasserstoff oder Fluor bedeuten.

3. Verfahren zur Herstellung der Verbindungen der Formel I, dadurch gekennzeichnet, daß man eine Verbindung der Formel II 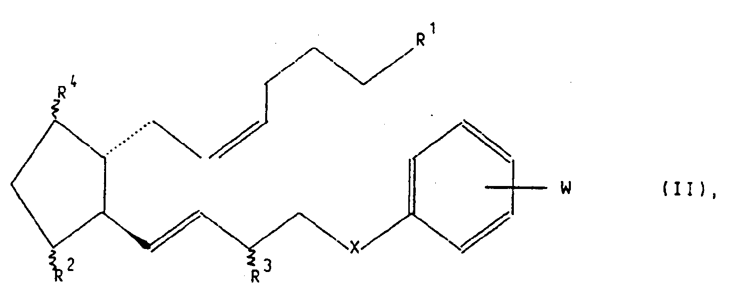 worin
R⁴ eine Hydroxygruppe und R¹, R², R³, X und W die oben angegebenen Bedeutun- gen aufweisen und freie OH-Gruppen in R², R³ und W geschützt sind, mit Diethylaminoschwefeltrifluorid oder anderen Fluorierungsmitteln umsetzt und geschützte Hydroxygruppen in R², R³ und W freisetzt und/oder freie Hydroxygruppen verestert, verethert und/oder eine veresterte Carboxygruppe verseift oder eine Carboxylgruppe mit einer physiologisch verträglichen Base in ein Salz überführt oder mit α-, β- oder γ-Cyclodextrin zu einem Clathrat umsetzt oder mit Liposomen verkapselt.

4. Arzneimittel aus einer oder mehreren Verbindungen nach Anspruch 1 und üblichen Hilfs-, Träger- und Zusatzstoffen.

5. Verwendung der 9-Fluor-prostaglandin-Derivate der Formel I nach Anspruch 1 als diagnostische Hilfsstoffe zur Charakterisierung von Prostaglandinrezeptoren und von TXA₂/PGH₂-Rezeptorsubtypen.

6. Verwendung der Verbindungen der Formel I zur Herstellung eines Arzneimittels zur Behandlung von Krankheiten, die auf einer Störung der TXA₂/PGH₂-Bildung beruhen.

## Claims

1. 9-fluoroprostaglandin derivatives of formula I 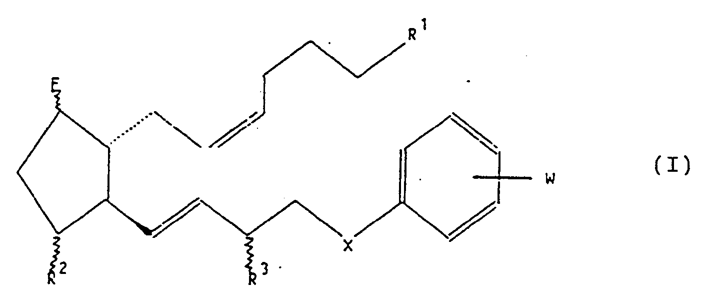 in which
R¹ may be 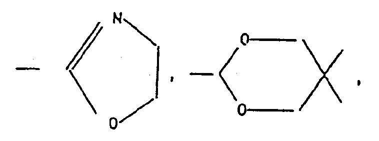 COOR⁴, wherein R⁴ may represent hydrogen or a C₁-C₁₀-alkyl radical that is optionally substituted by halogen atoms, hydroxy, C₁₋₄ alkoxy, C₆₋₁₂ aryl, which can be substituted by halogen, di-(C₁-C₄)-alkylamines or tri-(C₁-C₄)-alkylammonium, a C₃-C₁₀-cycloalkyl radical that is optionally substituted by C₁-C₄-alkyl, a C₇-C₁₆-aralkyl radical, a phenacycl radical substituted by W, a C₆-C₁₂-aryl which can be substituted by halogen atoms, phenyl, C₁-C₄-alkyl, chloromethyl, fluoromethyl, carboxyl, C₁-C₄-alkoxy or hydroxy or a 5- or 6-membered heterocyclic radical having at least one N, O or S atom, or R¹ may be a CONHR⁵ radical wherein R⁵ represents hydrogen, C₁-C₁₀-alkanoyl or C₁-C₁₀-alkanesulphonyl,
each of R² and R³ represents a hydrogen atom or a free or functionally modified hydroxy group, it being possible in each case for the OH group to be in the α- or β-configuration,
X may be a CH₂ group, an O atom or an S atom,
W may be hydrogen, -OR⁶, halogen, -CN-, -NO₂, trifluoromethyl or COOR⁶, and
R⁶ may be hydrogen, C₁-C₁₀-alkyl, that is optionally substituted by halogen atoms, hydroxy, C₁₋₄ alkoxy, C₆₋₁₂ aryl, which can be substituted by halogen, di-(C₁-C₄)-alkylamines or tri-(C₁-C₄)-alkylammonium, C₆-C₁₂-aryl which can be substituted by halogen atoms, phenyl, C₁-C₄-alkyl, chloromethyl, fluoromethyl, carboxyl, C₁-C₄-alkoxy or hydroxy or C₇-C₁₆-aralkyl,
and, when R⁴ represents hydrogen, the salts thereof with physiologically tolerable bases, as well as the α-, β- or γ-cyclodextrin clathrates, as well as the compounds of formula I encapsulated with liposomes.

2. 9-fluoroprostaglandin derivatives of formula I according to claim 1, characterised in that
R¹ represents the radical COOR⁴ wherein R⁴ is hydrogen or C₁-C₆-alkyl, or R¹ represents the radical CONHR⁵ wherein R⁵ is methylsulphonyl,
X represents a CH₂ group or an O atom,
R² and R³ represent hydrogen or hydroxy, and
W represents hydrogen or fluorine.

3. Process for the preparation of the compounds of formula I, characterised in that a compound of formula II 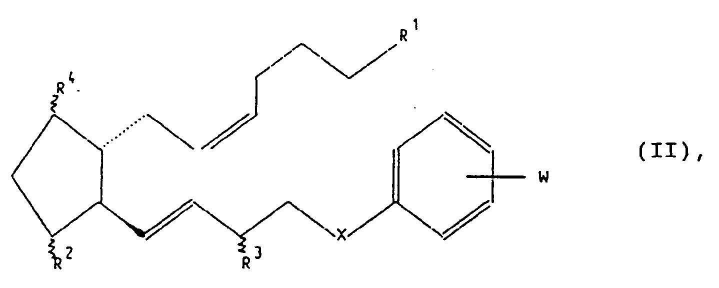 in which R⁴ represents a hydroxy group and R¹, R², R³, X and W have the meanings given above and free OH groups in R², R³ and W are protected, is reacted with diethylaminosulphur trifluoride or other fluorinating agents and protected hydroxy groups in R², R³ and W are freed and/or free hydroxy groups are esterified or etherified and/or an esterified carboxy group is hydrolysed or a carboxy group is converted into a salt with a physiologically tolerable base or is reacted with α-, β- or γ-cyclodextrin to form a clathrate or is encapsulated with liposomes.

4. Medicament comprising one or more compounds according to claim 1 and customary adjuvants, carriers and additives.

5. Use of the 9-fluoroprostaglandin derivatives of formula I according to claim 1 as diagnostic auxiliary agents for the characterisation of prostaglandin receptors and of TXA₂/PGH₂ receptor sub-types.

6. Use of the compounds of formula I for the preparation of a medicament for the treatment of disorders based on a disturbance in TXA₂/PGH₂ formation.

## Revendications

1. 9-Fluoro-prostaglandines de formule I ci-dessous : 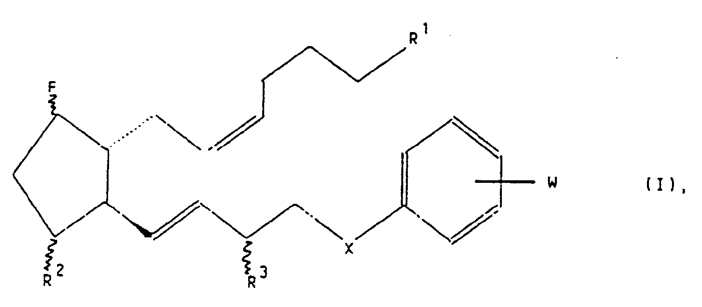 dans laquelle
R¹ représente un groupe 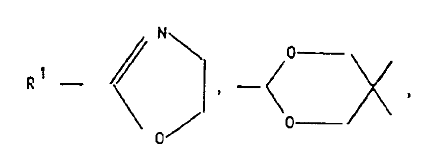 COOR⁴ R⁴ étant l'hydrogène
ou un alkyle en C₁-C₁₀ éventuellement substitué par halogènes, groupes hydroxyliques, alcoxy en C₁-C₄, aryles en C₆-C₁₂, pouvant avoir halogenes, di(C₁-C₄)alkylamines ou tri(C₁-C₄)alkylammonium, un cycloalkyle en C₃-C₁₀, éventuellement substitué par des alkyles en C₁-C₄, un aralkyle en C₇-C₁₆, un phenacyle substitué par W,un aryle en C₆-C₁₂ éventuellement substitué par halogenes, phenyle, alkyles en C₁-C₄, chloromethyle, fluoromethyle, carboxyle, alkoxy en C₁-C₄ ou hydroxy ou bien un radical hétérocyclique pentagonal ou hexagonal avec au moins un atome d'azote, d'oxygène ou de soufre, ou encore R¹ est un radical CONHR⁵, R⁵ pouvant être l'hydrogène ou bien un alcanoyle ou un alcane-sulfonyle en C₁-C₁₀,
R² et R³ représentent chacun un atome d'hydrogène ou un hydroxyle libre ou modifié, le groupe OH pouvant être à la position α ou β,
X désigne un groupe CH₂ ou un atome d'oxygène ou de soufre et
W l'hydrogène, -OR⁶, un halogène, ou un groupe -CN, -NO₂, trifluorométhyle ou COOR⁶,
R⁶ étant l'hydrogene, un alkyle en C₁-C₁₀ éventuellement substitué par halogènes, groupes hydroxyliques, alcoxy en C₁-C₄, aryles en C₆-C₁₂, pouvant avoir halogènes, de di(C₁-C₄)alkylamines ou tri(C₁-C₄)alkylammonium, un aryle en C₆-C₁₂ éventuellement substitué par d'halogènes, phenyle, alkyles en C₁-C₄, chloromethyle, fluoromethyle, carboxyle, alkoxy en C₁-C₄ ou hydroxy ou un aralkyle en C₇-C₁₆ ainsi que, si R⁴ est l'hydrogène, les sels de ces composés avec des bases physiologiquement compatibles et tolérées, ainsi que leur clathrates formés avec la cyclodextrine α, β ou γ, et les composés de formule I encapsulés avec des liposomes.

2. 9-Fluoro-prostaglandines de formule I selon la revendication 1, caractérisées en ce que
R¹ est le groupe COOR⁴, R⁴ étant l'hydrogène ou un alkyle en C₁-C₆, ou bien R¹ est le radical CONHR⁵, R⁵ étant un groupe méthylsulfonyle,
X est un groupe CH₂ ou un aotme d'oxygène,
R² et R³ sont l'hydrogène ou le groupe hydroxy, et
W désigne l'hydrogène ou le fluor.

3. Procédé de préparation des composés de formule I selon la revendication 1, procédé caractérisé en ce que l'on fait réagir un composé de formule II 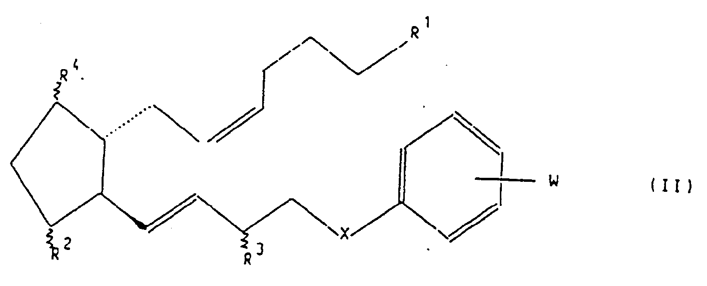 R⁴ étant un groupe hydroxy et les autres symboles ayant les significations indiquées, et des groupes OH libres de R², R³ et W étant protégés, avec du trifluorure de diéthylamino-soufre ou d'autres agents de fluoration, et on libère des groupes hydroxyliques protégés de R², R³ et W et/ou on estéréfie ou éthérifie des groupes hydroxyliques libres et/ou on saponifie un groupe carboxylique estérifié ou on salifie un groupe carboxylique avec une base physiologiquement compatible et tolérée ou encore on forme un clathrate par réaction avec la cyclodextrine α, β ou γ ou on encapsule le produit avec des liposomes.

4. Médicaments d'un ou de plusieurs composés de la revendication 1 avec des adjuvants, supports et additifs courants.

5. L'emploi des 9-fluoro-prostaglandines de formule I selon la revendication 1 comme agents auxiliaires de diagnostics pour caractériser des récepteurs de prostaglandines ou des sous-types de récepteurs de TXA₂/PGH₂.

6. L'emploi des composés de formule I pour préparer des médicaments en vue du traitement de maladies qui sont dues à une perturbation de la formation de TXA₂/PGH₂.
